# EUROPÄISCHE PATENTSCHRIFT

(11) **EP 2 088 885 B1**
(45) Veröffentlichungstag und Bekanntmachung des Hinweises auf die Patenterteilung: **29.09.2010**
(21) Anmeldenummer: 07822577.8
(22) Anmeldetag: 14.11.2007
(51) Int. Cl.: A43B 7/36, A61N 1/14

(54) **SCHUH MIT EINER ELEKTRISCH LEITFÄHIGEN SOHLE**
SHOE WITH AN ELECTRICALLY CONDUCTIVE SOLE
CHAUSSURE PRÉSENTANT UNE SEMELLE ÉLECTROCONDUCTRICE

(30) Priorität: 15.11.2006 AT 18912006
(43) Veröffentlichungstag der Anmeldung: 19.08.2009
(73) Patentinhaber: Wiebecke, Adolf, 5411 OBERALM (AT)
(72) Erfinder: Wiebecke, Adolf, 5411 OBERALM (AT)
(74) Vertreter: Babeluk, Michael
(86) Internationale Anmeldenummer: PCT/EP2007/062317
(87) Internationale Veröffentlichungsnummer: WO 2008/058984

(56) Entgegenhaltungen:
- EP-A- 0 791 302
- WO-A-96/41550
- FR-A- 2 287 244
- FR-A- 2 524 275
- GB-A- 2 288 981

## Beschreibung

Die Erfindung betrifft einen Schuh mit einer elektrisch leitfähigen Sohle gemäß dem Oberbegriff von Patentanspruch 1.

Es ist bekannt, dass der gesundheitliche Zustand und das Wohlbefinden von Menschen dadurch gesteigert werden können, dass eine elektrische Verbindung zwischen den Füßen und der Bewegung hergestellt wird. Auf diese Weise kann auch beim Tragen von Schuhwerk ein Zustand erreicht werden, der etwa dem des Barfußgehens entspricht. So zeigen etwa die DE 102 55 408 A, die WO 96/41550 A oder die DE 21 47 904 A Schuhe, bei denen Mittel vorgesehen sind, um einen elektrischen Kontakt zwischen den Fußsohle und der Schuhsohle herzustellen. Darüber hinaus sind aus der GB 2 288 981 A Schuhe mit Magneten bekannt, die in die Sohle eingearbeitet sind. In der Praxis hat sich diese Lösung als unzureichend herausgestellt.

Obgleich mit diesen bekannten Schuhen eine wesentliche Verbesserung gegenüber herkömmlichem Schuhwerk erreicht werden kann, sind die vorteilhaften Wirkungen beschränkt. Es können zwar negative Einflüsse weitgehend ausgeglichen werden, die Erzielung von positiven, die Gesundheit fördernden Wirkungen, kann jedoch mit solchen Schuhen nicht erreicht werden.

Aufgabe der vorliegenden Erfindung ist es, diese Nachteile zu überwinden und einen Schuh anzugeben, der einen nachweisbaren positiven Einfluss auf das Wohlbefinden und die Gesundheit des Trägers ausübt.

Erfindungsgemäß werden diese Aufgaben durch die Merkmale von Patentanspruch 1 gelöst. Wichtig ist, dass mindestens ein Permanentmagnet im mittleren Abschnitt der Sohle vorgesehen ist und einem Akupunkturpunkt zugeordnet ist. Durch die Kombination der elektrischen Erdung mit einem Permanentmagnet, der das Erdmagnetfeld überlagert und einen konstanten Einfluss auf die den Schuh tragende Person ausübt, können nachweisbare und überprüfbare Effekte erzielt werden. Diese Effekte sind dann von besonderer Signifikanz, wenn der Permanentmagnet im Bereich des Endpunktes des Nieren-Meridians angeordnet ist. Dieser Endpunkt, der Akupunkturpunkt N1, der auch als Yong Quan, "Sprudelnden Quelle" bezeichnet wird, ist an der Fußsohle an der Grenze vom vorderen zum mittleren Drittel hinter dem zweiten und dritten Metatarsophalangialgelenk in jener Vertiefung angeordnet, die bei Plantarflexion entsteht. Indikationen, die diesem Akupunkturpunkt zugeordnet werden, umfassen unter anderem Kopfschmerzen auf der Scheitelhöhe, Bewusstlosigkeit, Sonnenstich, Hysterie, Epilepsie, Haemorrhagia Cerebri, Miktionsstörungen.

Generell handelt es sich bei den Akupunkturmeridianen nach Vorstellungen der traditionellen chinesischen Medizin (TCM) um Leitungsbahnen, in denen das Qi, die sogenannten Lebensenergie, fließt. Einer genaueren Definition zu Folge handelt es sich um gedachte Verbindungswege des Energieaustauschs zwischen Körperoberfläche - und damit der Außenwelt - und den im körperinneren Orbes (=organische Funktionskreise). Es sind daher logische, aber nicht stoffliche Bahnen.

Die einzelnen Meridiane sind verschiedenen Organsystemen zugeordnet, so der konkrete Nieren-Meridian der Niere. Dabei ist jedoch festzustellen, dass der chinesische Organbegriff nicht mit dem der westlichen Medizin übereinstimmt. Die TCM verbindet den Begriff "Niere" - SHEN nicht nur mit Harnproduktion und Ausscheidung, sondern auch mit dem wesentlichen Bewahrer für Lebenskraft, Aktivität und Wachstumskraft. Der Niere auch eine Verbindung zum Ohr zugeschrieben, d.h., dass beispielsweise Schwerhörigkeit auf eine Nierenschwäche zurückgeführt wird. Ebenso folgt aus der Niere als Sitz der Lebensenergie die Zuordnung von Schwächegefühl, erhöhter Müdigkeit, leichter Erschöpfbarkeit, Antriebsmangel, Ängstlichkeit, gedrückter Stimmungslage und dergleichen, aber auch von Symptomen des Bewegungsapparates, wie etwa Steifigkeit der Wirbelsäule zu einer Nierenschwäche. Der Nieren-Meridian beginnt mit dem oben beschriebenen Punkt N1, verläuft dann unter die mediale Seite des Fußgelenks, des Unter- und des Oberschenkels, die Leistenbeuge und das Abdomen und endet im Punkt N27 Yu Fu (Werkstatt der Zustimmung) am Unterrand der Clavicula am Sternum-Ansatz.

Zur Evaluierung der Wirkung der Erfindung ist das an der staatlichen Universität St. Petersburg entwickelte Verfahren in Korotkov K.: Human Energy Field Study with GDV Bioelectrography. Backbone Publishing, St. Petersburg, 348 Seiten, 2003; Dobson P. und O'Keefe E.: Int. J. Altern. Complim. Med. 7, 12-17, 2000; Hacker et al., Forsch. Kompl. Med. Forsch. Komplementärmed. 12: 315-327, 2005 beschrieben worden.

Das in der vorliegenden Pilotuntersuchung verwendete Verfahren liefert mit der "Area-of-Glow" einen gut reproduzierbaren physikalisch-biomedizinischen Parameter für Analysen der "relativen Wirksamkeit verschiedener Stress-Interventionen" (Korotkov, 2002). In den letzten Jahren kristallisierte sich immer mehr heraus, dass der Area-of-Glow-Parameter die sensitive Anzeige bestimmter Formen von Stress erlauben würde. Insbesondere kann man aus dem Vergleich einer "Kontrolle" mit der Applikation eines möglichen "Stressors" herausfinden, ob eine relative Stärkung oder eine relative Schwächung des menschlichen Energiefeldes (Human Energy Field, HEF) vorliegen dürfte. Komplementärmedizinische Meridiananalysen von GDV-Coronalbildern ergeben in weiterer Folge auch Hinweise darüber, welche Organsysteme in welcher Art beeinflusst werden könnten.

Der Ablauf der GDV-Messungen ist wie folgt zu beschreiben:
Coronaentladungen an den zehn Fingerkuppen beider Hände wurden im Anschluss an die beiden jeweils 10-minütigen Testperioden jeweils 6 mal mittels der hochsensitiven GDV-Camera aufgezeichnet, mit Hilfe von Bild-zytometrischen Verfahren (Computer-Aided Image Analysis) ausgewertet und biostatistisch analysiert. Zusätzlich zu diesen "statischen GDV-Messungen" wurden an den Ringfingern beider Hände auch "dynamische GDV-Messungen" jeweils (2x3x20 Sekunden) durchgeführt.

Außerdem wurden, wie oben bereits angedeutet, aus den individuellen Verteilungen der "Corona"-Entladungen aus den unterschiedlichen Fingerkuppen nach den Prinzipien der Energiemerdianlehre und der Akupunktur komplementärmedizinisch auch "Ganzkörper-Coronadiagramme" ermittelt. Koeffizienten, mit denen die energetischen Zustände der jeweiligen Organe, bzw. Organsysteme charakterisiert werden können. Deren zahlenmäßige Abweichungen vom "optimalen Mittel" wurden ebenfalls statistischen Prüfungen zugeführt.

Die GDV-Analyse ist an den Händen der Versuchspersonen durchgeführt worden, da es aus dem Fachgebiet der Handakupunktur bekannt ist, dass die Akupunkturmeridiane und deren Akupunkturpunkte eine Entsprechung an den Händen haben. Im konkreten wird der Nierenmeridian in diesem System über die Mittelfinger und kleinen Finger beider Hände abgebildet. Dabei sind insbesondere folgende Punkte von Interesse:
- 3L2 - Linke Niere / repräsentiert auf der Kuppe des linken Mittelfingers, Segment 2
- 5L2 - Linke Niere / repräsentiert auf der Kuppe des kleinen Fingers der linken Hand, Segment 2
- 3R5 - Rechte Niere / repräsentiert auf der Kuppe des rechten Mittelfingers, Segment 5
- 5R4 - Rechte Niere / repräsentiert auf der Kuppe des kleinen Fingers der rechten Hand, Segment 4

Weiters wurde versucht, aus den GDV-Daten der Studie herauszufinden, ob gewisse energetische Effekte der erfindungsgemäßen Schuhe auf das Immunsystem und das Herz-Kreislauf-System feststellbar sind, wobei ebenfalls komplementärmedizinisch nach dem vom TCM abgeleiteten System gearbeitet worden ist. Folgende Fingerkuppenareale stehen für die oben angeführten Systeme:
- 3RL+3L5 - Immunsystem / repräsentiert auf den Kuppen der beiden Mittelfinger, Segmente 2 und 5
- 3R6+3L1 - Herz-Kreislauf-System / repräsentiert auf den Kuppen der beiden Mittelfinger, Segmente 6 und 1

Zunächst wurden Coronadiagramme in Form von Ganzkörperprojektionen aus den Messdaten berechnet und gezeichnet. Dabei hat sich für die erfindungsgemäßen Schuhe herausgestellt, dass im Vergleich zum Versuchen mit Schuhen nach dem Stand der Technik eine Glättung dieser Coronadiagramme erzielt worden ist. Dies bedeutet, dass meridianal angezeigte energetische Schwachstellen beim Stand der Technik oftmals deutlich abgemildert werden konnten.

Danach wurden die an den Fingerkuppen gemessenen Messdaten mathematisch statistisch ausgewertet. Zunächst wurde für alle 10 Fingerkuppen der GDV-"Area-of-Glow-Parameter" bestimmt. Dieser "Area-of-Glow-Parameter" wird in der Fachliteratur generell mit dem Vorliegen von Stress in Verbindung gebracht, wobei eine Erhöhung dieses Parameters einer Reduktion von Stress gleichgesetzt wird. Eine erhöhter GDV-"Area-of-Glow-Parameter" ist also ein Anzeichen einer Verbesserung des Wohlbefindens, bzw. Gesundheitszustandes.

Bei den durchgeführten Versuchen wurde mit dem Schuh nach dem Stand der Technik ein Mittelwert des "Area-of-Glow-Parameters" von 11.464 erzielt. Im Gegensatz dazu lag der Mittelwert bei den Untersuchungen mit erfindungsgemäßen Schuhen bei 12.879. Eine statistische Auswertung zeigt, dass die statistische Signifikanz des erhöhten "Area-of-Glow-Parameters" bei über 99.9% liegt.

Auch für Einzelbewertungen in den Punkte 3L2, 5L2, 3R5, 5R4 hat der erfindungsgemäße Schuh stets statistisch signifikant besser abgeschnitten.

Die Autoren der Studie, Univ. Prof. Dr. Gerhard W. Hacker und HR Prim. Univ. Prof. Dr. Gernot Pauser, kommen daher zum Schluss, dass das Tragen des erfindungsgemäßen Schuhs signifikante Auswirkungen auf den Gesundheitszustand, bzw. auf das Wohlbefinden der Versuchspersonen haben.

Eine besonders bevorzugte Ausführungsvariante der Erfindung sieht vor, dass die Sohle aus einer elektrisch isolierenden Schicht besteht, an deren Unterseite eine elektrisch leitfähige Schicht aufgebracht ist und dass ein Verbindungskabel vorgesehen ist, das die elektrisch leitfähige Schicht mit der Kontaktelektrode verbindet. Insbesondere ist es dabei von Vorteil, wenn das Verbindungskabel Silberfäden aufweist, die vorzugsweise geflochten sind. Es hat sich herausgestellt, dass auf diese Weise eine besonders hohe Wirksamkeit gegeben ist.

Wesentlich an der Erfindung ist weiters, dass der Permanentmagnet über eine Verbindungsleitung mit der elektrisch leitfähigen Sohle in Verbindung steht. Auf diese Weise wird sichergestellt, dass der Magnet das gleiche elektrische Potenzial wie die Sohle aufweist.

Eine optimale Ableitung der elektrischen Potenziale aus dem menschlichen Körper kann dadurch erreicht werden, dass die Kontaktelektrode als elektrisch leitfähiges Gewebe mit metallischen Fäden ausgebildet ist. Auf diese Weise können auch herkömmliche Socken getragen werden, ohne die Wirkung des Schuhs zu beeinträchtigen.

Ideal im Sinne der Erfindung ist es, wenn die Kontaktelektrode im Fersenbereich des Schuhs angeordnet ist.

Besonders günstig ist es, wenn die elektrisch leitfähige Sohle wellenförmig strukturiert ist. Es kann sich dabei um ein spezielles Wellblech handeln, das aus einer geeigneten Legierung, wie etwa auf Aluminiumbasis mit Zusätzen weiterer Metalle, wie etwa Gold, aufgebaut ist. Die spezielle Form enthält Berge und Täler, deren Abstände und Höhen in definiertem Verhältnis zueinander stehen und dem Zweck entsprechend angepasst werden können.

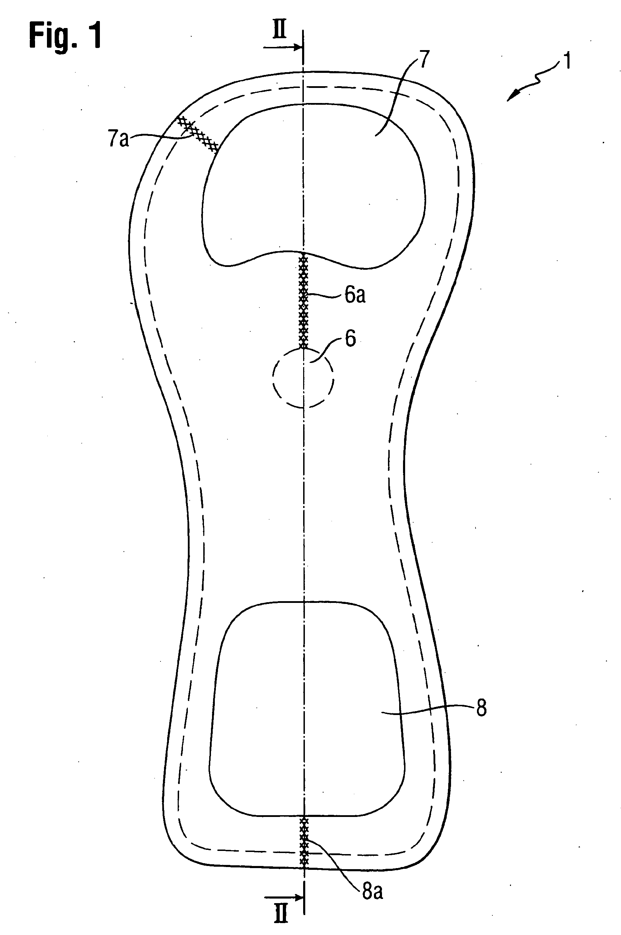

In der Folge wird die vorliegende Erfindung anhand des in den Figuren dargestellten Ausführungsbeispiels näher erläutert. Es zeigen
- Fig. 1: einen erfindungsgemäßen Schuh in einer Draufsicht und
- Fig. 2: einen Schnitt nach Linie II-II in Fig. 1.

Der in den Figuren dargestellte Schuh ist allgemein mit 1 bezeichnet. Der Schuh weist an seiner Unterseite eine Sohle 2 auf, die aus einer oberen isolierenden Schicht 3 und eine unteren leitfähigen Schicht 4 zusammengesetzt ist. An der Sohle 2 ist in herkömmlicher Weise der Oberteil 5 des Schuhs 1 befestigt.

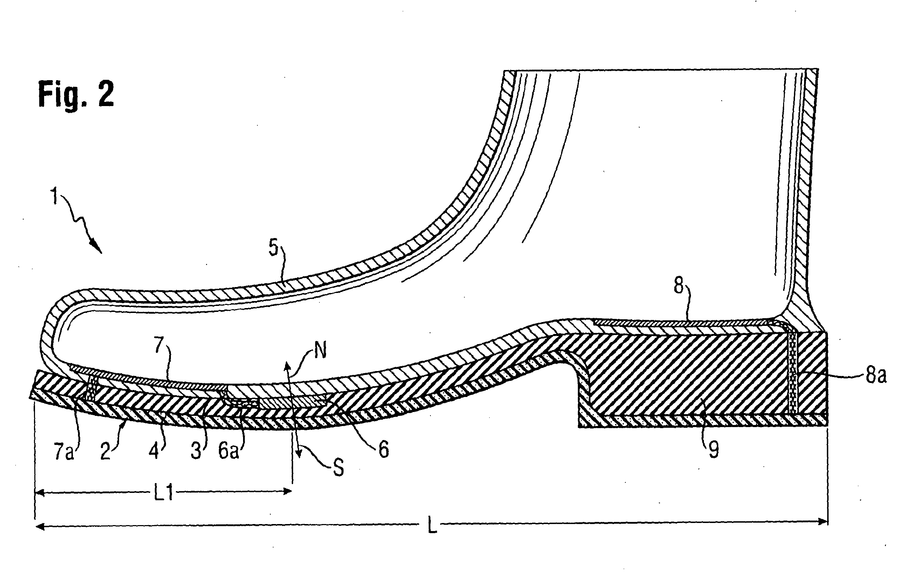

Im vorderen Drittel der Sohle 2 ist ein Permanentmagnet 6 in Form einer Scheibe eingearbeitet, dessen Nord-Süd-Richtung mit dem Doppelpfeil N-S in Fig. 2 eingetragen ist. Im Bereich des Fußballens und der Zehen ist eine erste Kontaktelektrode 7 vorgesehen, die über eine elektrische Leitung 7a aus geflochtenen Silberfäden mit der leitfähigen Schicht 4 der Sohle 2 verbunden ist. Im Fersenabschnitt des Schuhs 1 ist eine zweite Kontaktelektrode 8 angeordnet, die über eine weitere elektrische Leitung 8a mit der elektrische leitfähigen Schicht 4 im Bereich des Absatzes 9 des Schuhs 1 in Verbindung steht. Eine Verbindungsleitung 6a ist vorgesehen, um den Permanentmagnet 6 mit der Kontaktelektrode 7 und damit mit der elektrisch leitfähigen Schicht 4 der Sohle 2 zu verbinden.

Der Permanentmagnet 6 ist in Längsrichtung etwa mittig so im Schuh 1 angeordnet, dass der Abstand L1 des Permanentmagnet 6 von der Vorderkante des Schuhs 1 etwa ein Drittel der Gesamtlänge L des Schuhs 1 beträgt. Die beiden Kontaktelektroden 7, 8 bestehen aus einem Gewebe, in das metallische Fäden eingearbeitet sind.

In die Sohle kann ein hier nicht näher dargestelltes Hologramm eingearbeitet werden, das spezielle Runen, Symbole oder andere Informationen enthält.

Mit der vorliegenden Erfindung ist es möglich, Gesundheit und Wohlbefinden von Personen signifikant zu verbessern.

## Patentansprüche

1. Schuh (1) mit einer elektrisch leitfähigen Sohle (2) und mit mindestens einer Kontaktelektrode (7, 8) im Inneren des Schuhs (1), die mit der Sohle (2) elektrisch verbunden ist, **dadurch gekennzeichnet, dass** mindestens ein Permanentmagnet (6) im mittleren Abschnitt der Sohle (2) vorgesehen ist und einem Akupunkturpunkt zugeordnet ist und dass der Permanentmagnet (6) über eine Verbindungsleitung (6a) mit der elektrisch leitfähigen Sohle (2) in Verbindung steht.

2. Schuh (1) nach Anspruch 1, **dadurch gekennzeichnet, dass** die Sohle (2) aus einer elektrisch isolierenden Schicht (3) besteht, an deren Unterseite eine elektrisch leitfähige Schicht (4) aufgebracht ist und dass ein Verbindungskabel (7a, 8a) vorgesehen ist, das die elektrisch leitfähige Schicht (4) mit der Kontaktelektrode (7, 8) verbindet.

3. Schuh (1) nach Anspruch 2, **dadurch gekennzeichnet, dass** das Verbindungskabel (7a, 8a) Silberfäden aufweist, die vorzugsweise geflochten sind.

4. Schuh (1) nach einem der Ansprüche 1 bis 3, **dadurch gekennzeichnet, dass** der Permanentmagnet (6) unter dem Endpunkt des Nieren-Meridians, nämlich dem Akupunkturpunkt N1 angeordnet ist.

5. Schuh (1) nach einem der Ansprüche 1 bis 4, **dadurch gekennzeichnet, dass** der Permanentmagnet (6) in die Sohle (2) eingebettet ist.

6. Schuh (1) nach einem der Ansprüche 1 bis 5, **dadurch gekennzeichnet, dass** die Kontaktelektrode (7, 8) als elektrisch leitfähiges Gewebe (4) mit metallischen Fäden ausgebildet ist.

7. Schuh (1) nach einem der Ansprüche 1 bis 6, **dadurch gekennzeichnet, dass** die Kontaktelektrode (7, 8) im Fersenbereich des Schuhs (1) angeordnet ist.

8. Schuh (1) nach einem der Ansprüche 1 bis 7, **dadurch gekennzeichnet, dass** die elektrisch leitfähige Sohle (2) wellenförmige Strukturen aufweist.

9. Schuh (1) nach einem der Ansprüche 1 bis 8, **dadurch gekennzeichnet, dass** an der Außenseite des Schuhs (1) ein Hologramm angeordnet ist, das Runen, Symbole oder andere Informationen enthält.

## Claims

1. Shoe (1) with an electrically conductive sole (2) and with at least one contact electrode (7, 8) inside the shoe (1), which is electrically connected to the sole (2), **characterised in that** at least one permanent magnet (6) is provided in the middle section of the sole (2) and corresponds to an acupuncture point and that the permanent magnet (6) is connected to the electrically conductive sole (2) via a connecting line (6a).

2. Shoe (1) according to claim 1, **characterised in that** the sole (2) consists of an electrically insulating layer (3) on whose underside an electrically conductive layer (4) is applied and that a connecting wire (7a, 8a) is provided, which connects the electrically conductive layer (4) to the contact electrode (7, 8).

3. Shoe (1) according to claim 2, **characterised in that** the connecting wire (7a, 8a) contains silver filaments, which preferably are braided.

4. Shoe (1) according to any of claims 1 to 3, **characterised in that** the permanent magnet (6) is placed under the end point of the kidney meridian, i.e. the acupuncture point N1.

5. Shoe (1) according to any of claims 1 to 4, **characterised in that** the permanent magnet (6) is imbedded in the sole (2).

6. Shoe (1) according to any of claims 1 to 5, **characterised in that** the contact electrode (7, 8) is configured as an electrically conductive fabric (4) with metallic filaments.

7. Shoe (1) according to any of claims 1 to 6, **characterised in that** the contact electrode (7, 8) is positioned in the heel area of the shoe (1).

8. Shoe (1) according to any of claims 1 to 7, **characterised in that** the electrically conductive sole (2) has a wave-shaped structure.

9. Shoe (1) according to any of claims 1 to 8, **characterised in that** a hologram is located on the outside of the shoe (1), which contains runes, symbols or other information.

## Revendications

1. Chaussure (1) équipée d'une semelle (2) électro-conductrice et d'au moins une électrode de contact (7, 8) à l'intérieur de la chaussure (1), cette électrode étant reliée électriquement à la semelle (2),
**caractérisée en ce que**
au moins un aimant permanent (6) est prévu dans le segment médian de la semelle (2) et il est associé à un point d'acuponcture, et
l'aimant permanent (6) est relié par une ligne de liaison (6a) à la semelle électro-conductrice (2).

2. Chaussure (1) selon la revendication 1,
**caractérisée en ce que**
la semelle (2) est formée d'une couche isolante électrique (3) dont la face inférieure porte une couche électro-conductrice (4), et
un câble de liaison (7a, 8a) relie la couche électro-conductrice (4) à l'électrode de contact (7, 8).

3. Chaussure (1) selon la revendication 2,
**caractérisée en ce que**
le câble de liaison (7a, 8a) comporte des fils en argent qui sont de préférence tressés.

4. Chaussure (1) selon l'une des revendications 1 à 3,
**caractérisée en ce que**
l'élément permanent (6) se trouve sous le point d'extrémité du méridien de Nieren à savoir le point d'acuponcture N1.

5. Chaussure (1) selon l'une des revendications 1 à 4,
**caractérisée en ce que**
l'élément permanent (6) est intégré dans la semelle (2).

6. Chaussure (1) selon l'une des revendications 1 à 5,
**caractérisée en ce que**
l'électrode de contact (7, 8) est réalisée sous la forme d'un tissu (4) électro-conducteur formé de fils métalliques.

7. Chaussure (1) selon l'une des revendications 1 à 6,
**caractérisée en ce que**
l'électrode de contact (7, 8) est installée dans la zone du talon de la chaussure (1).

8. Chaussure (1) selon l'une des revendications 1 à 7,
**caractérisée en ce que**
la semelle électro-conductrice (2) a des structures de formes ondulées.

9. Chaussure (1) selon l'une des revendications 1 à 8,
**caractérisée en ce que**
le côté extérieur de la semelle (1) porte un hologramme contenant des runes, des symboles ou autres informations.
